(19) Europäisches Patentamt
European Patent Office
Office européen des brevets

(11) EP 2 292 740 B1

(12) # EUROPEAN PATENT SPECIFICATION

(45) Date of publication and mention of the grant of the patent:
**26.04.2017 Bulletin 2017/17**

(51) Int Cl.:
*C12N 7/00* (2006.01) *A61K 35/76* (2015.01)

(21) Application number: **10172708.9**

(22) Date of filing: **12.08.2010**

(54) **Phage of Acinetobacter baumannii**

Acinetobacter baumannii-Phage

Phage d'Acinetobacter baumannii

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **12.08.2009 TW 098127069**
**12.08.2009 TW 098127070**

(43) Date of publication of application:
**09.03.2011 Bulletin 2011/10**

(73) Proprietor: **Hualien Tzu Chi Hospital, Buddhist Tzu Chi Medical Foundation**
**Hualien County 97002 (TW)**

(72) Inventors:
- **Chen, Li-Kuang**
  **Hualien (TW)**
- **Lin, Nien-Tsung**
  **Hualien (TW)**

(74) Representative: **Epping - Hermann - Fischer Patentanwaltsgesellschaft mbH**
**Schloßschmidstraße 5**
**80639 München (DE)**

(56) References cited:
**WO-A2-2009/072762**

- **LIN N T ET AL: "Isolation and characterization of phiAB2: a novel bacteriophage of Acinetobacter baumannii" RESEARCH IN MICROBIOLOGY, ELSEVIER, AMSTERDAM, NL LNKD-DOI:10.1016/J.RESMIC.2010.03.007, vol. 161, no. 4, 1 May 2010 (2010-05-01), pages 308-314, XP027063637 ISSN: 0923-2508 [retrieved on 2010-04-10] -& DATABASE EMBL [Online] 9 July 2010 (2010-07-09), "Acinetobacter phage phiAB2 putative scaffolding protein (psf24) gene, partial cds." XP002609107 retrieved from EBI Database accession no. GU979514**
- **YANG HONGJIANG ET AL: "Isolation and Characterization of a Virulent Bacteriophage AB1 of Acinetobacter baumannii" BMC MICROBIOLOGY, vol. 10, April 2010 (2010-04), XP002609106 ISSN: 1471-2180**
- **SHEN G-H ET AL: "Isolation and characterization of lytic phages against pandrug resistant Acinetobacter baumannii" ABSTRACTS OF THE GENERAL MEETING OF THE AMERICAN SOCIETY FOR MICROBIOLOGY, AMERICAN SOCIETY FOR MICROBIOLOGY, WASHINGTON, US, vol. 106, 1 January 2006 (2006-01-01), page 369, XP009140997 ISSN: 0067-2777**
- **SOOTHILL J S: "TREATMENT OF EXPERIMENTAL INFECTIONS OF MICE WITH BACTERIOPHAGES" JOURNAL OF MEDICAL MICROBIOLOGY, HARLOW, GB, vol. 37, no. 4, 1 January 1992 (1992-01-01), pages 258-261, XP009009001 ISSN: 0022-2615**
- **MATSUZAKI SHIGENOBU ET AL: "Bacteriophage therapy: a revitalized therapy against bacterial infectious diseases" JOURNAL OF INFECTION AND CHEMOTHERAPY, vol. 11, no. 5, October 2005 (2005-10), pages 211-219, XP002609108 ISSN: 1341-321X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 292 740 B1

- HANLON ET AL: "Bacteriophages: an appraisal of their role in the treatment of bacterial infections" INTERNATIONAL JOURNAL OF ANTIMICROBIAL AGENTS, ELSEVIER SCIENCE, AMSTERDAM, NL LNKD- DOI:10.1016/J.IJANTIMICAG.2007.04.006, vol. 30, no. 2, 27 June 2007 (2007-06-27), pages 118-128, XP022132522 ISSN: 0924-8579
- ACKERMANN H -W ET AL: "Classification of Acinetobacter phages" ARCHIVES OF VIROLOGY, vol. 135, no. 3-4, 1994, pages 345-354, XP002609109 ISSN: 0304-8608
- JOLY-GUILLOU M L ET AL: "A study of the relationships between antibiotic resistance phenotypes, phage-typing and biotyping of 117 clinical isolates of Acinetobacter spp" JOURNAL OF HOSPITAL INFECTION, ACADEMIC PRESS, LONDON, GB LNKD- DOI:10.1016/0195-6701(90)90048-S, vol. 16, no. 1, 1 July 1990 (1990-07-01), pages 49-58, XP023273111 ISSN: 0195-6701 [retrieved on 1990-07-01]

SEQUENCE LISTING

<110> Buddhist Tzu Chi General Hospital

<120> Phage of *Acinetobacter baumannii*

<130>

<160> 4

<170> PatentIn version 3.3

<210> 1
<211> 2412
<212> DNA
<213> Phage of *Acinetobacter baumannii*

<220>
<221> gene
<222> 1..1961
<223> RNA polymerase

<400> 1

atgtctgatc tataccaacg ccaaatagct cttgaagaat catacagtca cgatagtatc 60

attgctggtc agaagcaagt actagatgca tatcaacaag gacgtgctgc tgacgtaggt 120

acaggtcgta tcctattagc taaagcattt gaagttggtg tagaagcttt aaatgcagtt 180

aagaaacaaa agattcgtgg tgttggcggt aaatacttaa aattactttc tatcgctgat 240

ccagaagttt tagtaatggc tgcattacgt gatattatta atgcatgtgc tgtacctgaa 300

ccagtgtcta tgcagaaagt acttacgggt attgggcgta tgattgagtc agagtctatg 360

ttggtattta tgcaggagtt aaatcctgca tacactgata agactattca gtatttagac 420

aacacaggta caaaatcagt tacccaccgt tatcgtacat tcttagcagg ttctaagtct 480

attcaactag attgggaaca gtggtcacaa gaggagcgta taggtgtagc taagttgttg 540

gtaagttgtt tatacgacgc tacaggatta ttccaatggg ctaaactgga tagcggtatg 600

taccacatta aagcttctga acccttagcg aagcactttc aggatgcagc gagtgcagcg 660

agagcagttg ttaaatatcc tcctatgttg atcaaaccta tggattggga aggacagtat 720

aacggtgggt atttaactga atggtttaaa cataactcac ctatgtgtgg tattcgcttt 780

attaagaaag agcataagca ttgggttatt gataacttaa acaatggtgc agagctagtt 840

aaggctgcaa tgaataaagc acagtctgta ccttacagga tcaataaaga catcttagca 900

atcttacgta aagcagttgc tatgcgtgta ggtattttag gtttaccaag ctatcaacct 960

gcaccgaaac ctgcatttcc atttactgat gattggttaa agtcagaagc tactgaggaa 1020

gaattagatc agttccaatt ctggaaaggt ttaatgagtt catggtatac acaagaagct 1080

aaacgtgttg gtcgtcaaca tggcatctta agtcgtattc aagaactggt taaatatcag 1140

gacgaagaac gtttgtactt tccaacgttt attgattggc gaggtcgtct ttacttccgt 1200

agtagtatta acccacaatc gaatgattgt attaaaggtt gtcttgagtt tgcagagggt 1260

aaacctctag gtaaaacagg acttaaatgg ttaaagattc atgttgcaaa ctgttgtggt 1320

tatgataaac atgatcctga tttaaaggag aaatggtgtg atgacaactg gaactacatt 1380

cagaatttta tcaataaccc gtttgatgtt gaagcacccg aacccgacac tgcttttaca 1440

ttactacaag caggtttggc gctacaatct gcgttggaat tggaaacacc tgaatcctac 1500

atatgccatg tccccgtcgc aatggacgca acttgttcgg gtctacagca cctatctgcg 1560

ctcactagag acgaagtagg tggtttatat acgaacttat tagacaacgg tgaagaccag 1620

aagtctgata tttatatgcg tgtagcgcac atagcagatg agtctaaact agaattagct 1680

gattctcctg ctgtacgtca gtattgggtg gataaaccta ttagtcgtaa tatggcgaag 1740

aaacctgtga tgacttacgt atacggttcg aagttattat caactattca aggcttagct 1800

aatgatatgt atgaagcagg tatggatgag attcagttag atggtaagac agtctttact 1860

tacaaccgat tagctaaacc agttggtaag gcattacgta aaggtgtcga agatactgta 1920

cctaaatctg ctgagatgat gaactacttg cagaacgttg tacgtaaaaa taaagctgat 1980

gctatgcgtt ggtttagtcc agtaggtgtt cctgttgtga attgggcaga aggtatggtg 2040

actaaacttg tagcaattcg ttcgatggga atctccagaa ttgcttatag ttatccagat 2100

aaccaatata ataccttaag agcagctaac ggtattgtac ctaactttgt acatagtatg 2160

gatagcagtc acttatgttt aactatctta gatttcgatg gtcaagttct accaattcat 2220

gactcattcg caacccatcc tagtgatgtg gaagctatgc atgtatcatt acgtaagaca 2280

ttcattgaaa tgtatacaca attcagtatt gaagacttct taaagtttaa caatattgat 2340

cttgaagagt acacaccacc acttacaggt aacttagagt tatcggaaat ttctaaatcc 2400

cgttatatgt tt
2412

<210> 2
<211> 792
<212> DNA
<213> Phage of *Acinetobacter baumannii*

<220>
<221> gene
<222> 1..669
<223> RNA polymerase

<400> 2

atccagaagt tttagtaatg gctgcattac gtgatattat taatgcatgt gctgtacctg 60

aaccagtgtc tatgcagaaa gtacttacgg gtattggtcg tatgattgag tcagagtcta 120

tgttggtatt tatgcaagag ttaaaccctg catatactga caagactatc cagtatttag 180

acaacacagg tacaaaatca gttacccacc gttatcgtac attcttagca ggttctaagt 240

ctattcaact agattgggaa cagtggtcac aagaggagcg tataggtgta gctaagttgt 300

tggtaagttg tttatacgac gctacaggat tattccaatg ggctaaactg gatagcggta 360

tgtaccacat taaagcttct gaacccttag agaagcactt tcaggatgca gcgagtgcag 420

cgagagcagt tgttaaatac ccgcctatgt tgatccaacc tatggattgg gaaggacagt 480

ataacggtgg atatttaact gaatggttta aacataactc acctatgtgt ggtattcgct 540

ttattaagaa agagcacaag cattgggtta ttgataactt aaacaatggt gcagagctag 600

ttaaggctgc aatgaataaa gcacagtctg taccttaccg tatcaataaa gacatcttag 660

caatcttacg taaagcagtt gctatacgtg taggtatttt aggtttacca agttaccaac 720

ctgcacctaa acctgcattt ccatttactg aggattggtt aaagtctgag gctactgagg 780

aagaattaga tc
792

<210> 3
<211> 1557
<212> DNA
<213> Phage of *Acinetobacter baumannii*

<220>
<221> gene
<222> 1..155)
<223> phage head-tail connector

<400> 3

tgaagtccaa aggaaatgat tttacaaaga ctattcgagc tttgtacgat gaatacacgg 60

acgattcttt aaaaacaaga ttagaaatgt atgcactttg gactctacct agcgtgttcc 120

cgacaggtga gattacggta gataatggaa atgccgagat tgagcatgac taccaaagtg 180

taggcgcata tctagtgaat cggttagcgt cacgtttagc gagtacgtta tttcccgtaa 240

gcacatcttt ctttagaatc gaacctagtc aagagttgaa agacttagtt gataaacgtg 300

gtacaagtac ccttatcgac ttagagaaca aagcttgtcg tcgtttattc ttcaacgcat 360

cttatgcaca gattgtgcaa gcactgcgtt tacttattat cactggtgaa gttttattac 420

ttcgtagaga taatcgccta cgtgttttta gtttaaagaa ttatgcgtta ctacgcaaca 480

atgtagggga agtacttgag atcatcacac gagaacctaa acgttatcgg gaattagatg 540

ctgagactca ggcactccta caagatcgta acgaggacga gacccttgat ctttatacta 600

gaatccgtaa gcgtaatatc aatggggtaa tctcatggaa gattacacaa gaaatagatg 660

gtgtacgctt accaaactat gaaatctacc gagataagtt atgcccatat attcctgtaa 720

cgtggagtta tatgaatggt gatgcttacg gtcgtggtta cgtagaagag tatgcaggtg 780

actttgctaa gttatctgaa ctctcacaag gtttaacaga gtaccagatc gagtcattaa 840

ttatccgtca tgtatataat gcacagggtg gttttgatgt agaatctgct gtgaactcac 900

gtaacggtga ttggattagt ggtaacgtta atgctgtaca gaactatgaa tctggatcat 960

atcaaaagat gaatgaggtt cgattaggtt tagaagctat tatgcaacgt ctaaacgtag 1020

cgttcatgta cacaggtaat atgcgagaag gtgatcgtgt aacagcctat gagattgcac 1080

gtaatgctga tgaagcagag caagtcctcg gtggtgtgta ctcacaacta tctcagaata 1140

tgcatttacc tttagcatat ctattactct atgaagttcg taaagacttt attcaggcga 1200

ttgatagaca agaaatcgaa ttaaatattc taactggttt acaagcatta tcacgtagtt 1260

cagagaacca agctttatta gtagcagcga atgagattgc tacagttgcc caagtattct 1320

cacaagtaag taaacgattt aatcttgatg ctattgtaga taagattcta ctttctaatg 1380

gtattgatat ttcagagatt acatacagtg aagaagaaat gagagctaag gctatggaag 1440

aacaacgtgc agcagaggca cagcgacaac aagtaataca acaagctggc gcacagttag 1500

gtggtaatca attagaaaat acacaggctg ctcaattggc agcaggtatt caatagg 1557

<210> 4
<211> 360
<212> DNA
<213> Phage of *Acinetobacter baumannii*

<220>
<221> gene
<222> (1)..(360)
<223> phage head-tail connector

<400> 4

tatactagaa tccgtaagcg taatatcaat ggagtaatct catggaagat tacacaagag 60

atagatggtg tacgtttacc aaactatgaa atctaccgag ataagttatg cccatatatt 120

cctgtaacgt ggagttatat gaatggtgat gcttacggtc gtggttacgt agaagagtat 180

gcaggtgact ttgctaagtt atctgaactc tcacaaggtt taacagagta ccagatcgag 240

tcattaatta tccgtcatgt atataatgca cagggtggtt ttgatgtaga atctgctgtg 300

aactcacgta acggtgattg gattagtggt aacgttaatg ctgtacagaa ctatgaatct 360

## Description

### 1. Field of Invention

The present invention relates to a novel phage, and more particularly, to a phage of *Acinetobacter baumannii.*

### 2. Background of the Invention

Nosocomial infections are tough issues in Hospitals. Generally, the nosocomial infection rate is about from 3% to 5%. Organisms causing nosocomial infections are usually opportunistic pathogens. In other words, these bacteria are not harmful to hosts with normal immunity, and some of them are even normal flora to human; however, while hosts have weak immunity, the bacteria cause infections, resulting in diseases.

Bacteria causing nosocomial infections may exist in stethoscopes, anamnesis papers, tourniquets, grooves, syringe needles, respirators, humidifiers, furniture, floors, vents, monitors, water, soil, food (fruits, vegetables), dirt in drainage, human body such as skin, armpits, mucosal, oral cavity, upper respiratory tract, nasal cavity, gastrointestinal tract, etc.

For example, nosocomial infections occur in an intensive care unit since patients in the intensive care unit have weak immunity and have invasive therapies such as being cannulated. According to statistics, the nosocomial infection rate in an intensive care unit is about from 2% to 3%.

Currently, the most common bacteria causing nosocomial infections include *Pseudomonas aeruginosa*, *Staphylococcus aureus*, *Acinetobacter baumannii*, etc.

Antibiotics are general therapeutic agents for treating bacterial infections. However, when an antibiotic is overused, bacteria will be selected to have resistance to more antibiotics. In current nosocomial infections, there are more and more bacteria having resistance to antibiotics, and patients infected by these bacteria have to be treated with expensive and novel antibiotics. Further, if the antibiotic resistance keeps developed, there will be no effective antibiotic for therapy.

*Acinetobacter baumannii* (abbreviated as AB, hereafter) belong to Gram negative bacteria. Generally, *Acinetobacter baumannii* exist in skin, respiratory tract, and gastrointestinal tract in 10% population of human. *Acinetobacter baumannii* favor warm and humid environment, so as to exist in medical devices, water troughs, beds, bed mats, respiratory devices and even air in a hospital. Currently, *Acinetobacter baumannii* having multiple resistances to gentamicin, amikacin piperacillin/tazobactam, ticarcillin/clavulanate, ceftazidime, cefepime, cefpirome aztreonam, imipenem, meropenem, ciprofloxacin and levofloxacin have been isolated. Since *Acinetobacter baumannii* easily become having multiple resistances and are capable of living for a while on surfaces of an object, it is a tough issue in prevention and treatment of nosocomial infections.

Phages (bacteriophages) are viruses that infect bacteria, and grow and replicate in bacteria. There are lytic phages and lysogenic phages. Lytic phages infect bacteria, replicate in bacteria, and then are released from bacteria by lysing and killing bacteria. Lysogenic phages are capable of undergoing lytic or lysogenic life cycles, and exist in host cells while in lysogenic life cycles.

It has been disclosed that bacterial diseases are treated with phages. For example, US5,688,501, US5,997,862, US6,248,324 and US6,485,902 have disclosed a pharmaceutical composition comprising phages for treating bacterial diseases, group A streptococcal infections, dermatological infections, and control of Escherichia coli 0157 infections, respectively. US6,121,036 has disclosed a pharmaceutical composition having at least one phage. US6,699,701 has disclosed using *Salmonella enteritidis*-specific phages for packing food, in which a package material is coated with phages, and food (such as fruit and vegetables) is packed with the package material.

Shen et al. discloses isolation and characterization of lytic phages against pandrug resistant Acinetobacter baumannii (Abstracts of the General Meeting of the American Society for Microbiology 106, 2006, page 369). Otherwise, there are no publications disclosing *Acinetobacter baumannii*-specific phages, which are used for reducing the population of *Acinetobacter baumannii* and further reducing nosocomial infections.

## SUMMARY OF THE INVENTION

The present invention provides an isolated *Acinetobacter baumannii* phage deposited in DSMZ (German Collection of Microorganisms and Cell Cultures, Germany) and having a deposition number DSM 23599. The present invention also provides an isolated *Acinetobacter baumannii* phage deposited in DSMZ (German Collection of Microorganisms and Cell Cultures, Germany) and having a deposition number DSM 23600. The present invention further provides variants of the DSM 23600 phage, each variant having one or more sequences selected from the group consisting of sequences having more than 80% homology to SEQ ID NO: 1 and, 2, and encoding an RNA polymerase of the *Acinetobacter baumannii* phage, and to SEQ ID NO: 3 and 4 and encoding a head-tail connector of the *Acinetobacter*

*baumannii* phage, and having a genomic sequence with a homology of more than 80% compared to the genomic sequence of the DSM 23600 phage.

It is known that the nucleotide sequence of RNA polymerase is a highly conserved region in viral genome, and thus homology among species can be determined by identifying homology of RNA polymerase. In the present invention, sequences of SEQ ID NO. 1 and SEQ ID NO. 2 are DNA sequences encoding RNA polymerase of *Acinetobacter baumannii* phages. Upon sequence alignment, there is no viral sequence in the gene bank identical or similar to the sequences of SEQ ID NO. 1 and SEQ ID NO. 2 in the present invention.

The *Acinetobacter baumannii* phages of the present invention are lytic phages and specifically infect *Acinetobacter baumannii.* In other words, after the phages of the present invention infect host cells, *Acinetobacter baumannii,* the phages replicate and propagate in the host cells and lyse cell walls of host cells, and then *Acinetobacter baumannii* are destructed along with the release of the phages. Accordingly, the phages of the present invention are capable of reducing the amount of *Acinetobacter baumannii* and disinfecting environments, especially reducing nosocomial infections caused by *Acinetobacter baumannii.*

In an aspect of the present invention, the *Acinetobacter baumannii* phages are capable of attaching rapidly to *Acinetobacter baumannii,* have short latent period, and have large burst size upon lysis of *Acinetobacter baumannii.*

The phages of the present invention have double-stranded DNA having 35 to 40 kb as genetic material. FIG. 1 shows the viral particles of the phage of the present invention, in which the viral particle has a head portion with 20 faces and a tail portion have filament structure for attaching to the surface of host cells. The head portion of the viral particle is about 60 nm, and the tail portion is about 9 to 11 nm.

In an aspect of the present invention, the *Acinetobacter baumannii* phages have acid tolerance and alkali tolerance, and have bioactivity in the environment at pH 4 to 12. In the present invention, the term "bioactivity" refers to that the pages are capable of infecting host cells, *Acinetobacter baumannii,* propagating in the host cells and/or lysing the host cells.

In an aspect of the present invention, the phages of the present invention have bioactivity in a surfactant.

In one embodiment of the present invention, the surfactant is one selected from the group consisting of an anionic surfactant, a cationic surfactant, an amphoteric surfactant and a non-ionic surfactant.

In one embodiment of the present invention, the anionic surfactant can be, but not limited to, ammonium dodecyl sulfate, disodium laureth sulfosuccinate, disodium octyl sulfosuccinate, linear dodecyl benzene sulfonates, dodecyl phosphates (mono alkyl phosphate, MAP), secondary alkane sulfates (SAS), sodium cocoyl isethionate (SCID), sodium lauryl ether sulfate (SLES), sodium lauroyl sarcosinate, sodium lauryl sulfate (SLS), sodium taurine cocoyl methyltaurate and so on.

In a preferred embodiment of the present invention, the cationic surfactant can be, but not limited to, cetyl trimethyl ammonium chloride, dicocodimonium chloride, didoctyl dimethyl ammonium chloride, diester quaternary ammonium salts, alkyl dimethyl benzyl ammonium chloride, ditallow dimethyl ammonium chloride (DTDMAC), imidazoline quaternary ammonium salts and so on.

In a preferred embodiment of the present invention, the amphoteric surfactant can be, but not limited to, cocoyl Imidazolinium betaine, cocoamidopropyl hydroxysultaine, cocpamidopropyl dimethyl betaine, disodium cocoamphodipropionate, lauramidopropyl betaine, sodium alkylamphopropionate, tallow dihydroxyethyl betaine and so on.

In a preferred embodiment of the present invention, the non-ionic surfactant can be, but not limited to, alkyl polygluoside (APG), cocoamide DEA, lauramine oxide, lauryl ether carboxylic acid, Triton X (such as TX-100, TX-405, etc.), PEG-150 di-stearate, Tween (such as Tween-40, Tween-80, etc.) and Span (such as Span-20, Span-80, etc.) and so on.

In a preferred embodiment of the present invention, the surfactant is a non-ionic surfactant.

In a preferred embodiment, the surfactant is a commercial product, especially a detergent.

The present invention provides *Acinetobacter baumannii* phages for sterilizing *Acinetobacter baumannii,* and for preparing a pharmaceutical composition for treating diseases caused by *Acinetobacter baumannii.* In an aspect of the present invention, *Acinetobacter baumannii* phages are used as a sterilizing agent in health care centers (such as home care nursing), medical centers (such as hospitals, sanitaria, etc.) and medical research institutes, so as to reduce the amount of *Acinetobacter baumannii* in the environment.

*Acinetobacter baumannii* phages of the present invention can be used in health care centers, medical centers and medical research institutes, for example, but not limited to, intensive care units, surgeries, recovery rooms, consulting rooms and conference rooms. Also, *Acinetobacter baumannii* phages of the present invention can be applied to equipments in hospitals and sanitaria, for example, but not limited to, stethoscopes, anamnesis papers, tourniquets, grooves, syringe needles, respirators, humidifiers, furniture, floors, vents and monitors.

In a preferred embodiment of the present invention, *Acinetobacter baumannii* phages of the present invention can be directly or indirectly sprayed or applied on the objects (such as lotion for human skin). Alternatively, the objects can be immersed in the composition having *Acinetobacter baumannii* phages of the present invention.

## BRIEFE DESCRIPTION OF THE DRAWINGS

FIG. 1 shows SEM images of *Acinetobacter baumanni* phages according to the present invention;
FIG. 2A shows DNA pulsed-field gel electrophoresis patterns of restriction digests of *Acinetobacter baumannii* phage according to one embodiment of the present invention, using short (0.2-12 s for 6.5 h, left panel) and long (0.2-0.5 s for 16.5 h, middle and right panel) running conditions, in which M is molecular standard, 1 to 9 respectively indicate DNA samples treated with HincII, HindIII, SnaBI, SspI, EcoRV, BglII, MluI, XbaI, and EcoRI;
FIG. 2B shows the restriction enzyme map of DNA of *Acinetobacter baumannii* phage according to one embodiment of the present invention;
FIG. 3 shows SDS-polyacrylamide gel electrophoresis of viron protein of *Acinetobacter baumannii* phage according to one embodiment of the present invention, in which M is molecular standard;
FIG. 4 shows the absorption of *Acinetobacter baumannii* phage according to the present invention to *Acinetobacter baumannii* ATCC17978;
FIG. 5 shows the one-step growth curve of *Acinetobacter baumannii* phages according to the present invention on *Acinetobacter baumannii* ATCC 17978;
FIG. 6 shows the viability of *Acinetobacter baumannii* phages according to the present invention in surfactants;
FIG. 7A shows the viability of *Acinetobacter baumannii* phages according to the present invention at different temperatures;
FIG. 7B shows the viability of *Acinetobacter baumannii* phages according to the present invention at different temperatures and thaw conditions;
FIG. 8 shows the viability of *Acinetobacter baumannii* phages according to the present invention at different pH; and
FIG. 9 shows the viability of *Acinetobacter baumannii* phages according to the present invention in chemicals.

## DETAILED DESCRIPTION OF THE PREFERED EMBODIMENTS

The detailed description of the present invention is illustrated by the following specific examples. Persons skilled in the art can conceive the other advantages and effects of the present invention based on the disclosure contained in the specification of the present invention.

Example 1: Isolation of *Acinetobacter baumannii* phages

There were 87 samples collected from washing solution of catheter, waste water from drainage systems and untreated waste water in Buddhist Tzu Chi General Hospital, Hualien (Taiwan). The samples were respectively centrifuged at 5,000x g (4 °C) for 10 minutes, and then the supernatants were filtered via filters of 0.45 $\mu$m for plaque tests.

10 $\mu$l of each filtrate was dropped to bacterial lawns (preparation method described in Example 2) of *Acinetobacter baumannii.* If there were phages in the filtrate, there would be clear zones on the bacterial lawns. Then, the clear zones were picked up and immersed in LB medium, which was filtered to remove bacteria, so as to obtain high concentrated phage solution. Subsequently, the concentrated phage solution was diluted, and plated on the LB plate to form plaques. Single plaque isolation process was performed for at least twice to obtain pure phages.

After identification, there were four strains of *Acinetobacter baumannii* phages obtained in the present invention, which were named as $\psi$AB1 (deposition number: DSM 23599), $\psi$AB2 (deposition number: DSM 23600), $\psi$AB3 (a variant of $\psi$AB2) and $\psi$AB4 (a variant of $\psi$AB2), wherein $\psi$AB3 and $\psi$AB4 respectively have more than 80% of homology to $\psi$AB2. The four strains of phages were all capable of infecting *Acinetobacter baumannii* with different infectivity.

Example 2: Tests of host cell specificity

In order to test the *Acinetobacter baumannii* specificity of phages obtained in the present invention, *Acinetobacter baumannii* strains listed in Table 1 were used, in which 35 *Acinetobacter baumannii* strains were collected from Buddhist Tzu Chi General Hospital, Hualien, and 2 strains were obtained from ATCC (American Type Culture Collection).

The bacteria were cultured in the LB medium (Difco Laboratories, Detroit, MI, USA) at 37°C, and the bacterial growth was monitored by turbidity at OD.600. When OD unit was 1, the bacterial concentration was $3\times10^8$ cells/ml. Bacterial lawns were prepared by covering 1.8% of LB agar plate with a layer of 0.7% of LB agar having host cells (strains as listed in Table 1).

10 $\mu$l of phage solution ($10^{10}$ PFU/ml) obtained from example 1 was dropped into the bacterial lawns. The agar plate was dried for 10 minutes in the laminar flow, and then incubated at 37°C for 18-20 hours. Subsequently, the production of plaques was observed.

Table 1

| Strain | Features | Source |
|---|---|---|
| *Acinetobacter baumannii* | | |
| 19606,17978 | ATCC standard strains | ATCC |
| M495, M1094, M2472, M2477, M2641, M2835, M3069, M3237, M3739, M3982, M4666, M5473, M67329, M67649, M67777, M68092, M68282, M68316, M68630 (deposition number: DSM 23587, a host of ψAB1), M68651, M68653, M68661, 45530, 46709, 47538, 47543, 48393, 48465, 48829, 49575, 50064, 50068, 50913, 51360 | Clinical strains, MDRAB | Buddhist Tzu Chi General Hospital, Hualien |
| $M2383^s$ | Clinical Strain | Buddhist Tzu Chi General Hospital, Hualien |
| Ab1-Ab9 | $Imi^r$ $Mer^r$ $Amp^r$ | Wu et al. (2007) |
| *Acinetobacter calcoaceticus* | | |
| 33305 | ATCC standard strain | ATCC |
| *Escherichia coli* | | |
| DH5α | *endA1 hsdR17 (rk- mk+) supE44 thi-1 recA1 gyrA relA1φ80d lacZΔM15Δ(lacZYA-argF)U169* | Hanahan D. (1983) |
| G0003, G0004, G0008, G0010, G0012, G0070, G0071, G0072, G0081 | Clinical strains | Buddhist Tzu Chi General Hospital, Hualien |
| *Klebsiella pneumoniae* | | |
| Kp2, Kp50, Kp53, Kp90, Kp120, Kp121 | Clinical strains | Wu et al. (2007) |
| *Pseudomonas aeruginosa* | | |
| Pa79, Pa81, Pa86 | Clinical strains | Wu et al. (2007) |
| MDRAB : *Acinetobacter baumannii* have multiple resistances to gentamicin, amikacin, piperacillin/tazobactam, ticarcillin/clavulanate, ceftazidime, cefepime, cefpirome, aztreonam, imipenem, meropenem, ciprofloxacin, and levofloxacin.<br>Amp: ampicillin; Imi: imipenem; Mer: meropenem; r: resistant; s: sensitive | | |

The phages obtained from example 1 formed no plaque on the bacterial lawns of *A. calcoaceticus*, 10 strains of *E. coli*, 6 strains of *K. pneumoniae* and 3 strains of *P. aeruginosa*, and plaques were only formed on the bacterial lawns of *Acinetobacter baumannii.* Hence, the phages of the present invention specifically infected *Acinetobacter baumannii.* The phages obtained from example 1 formed plaques on the bacterial lawn of *Acinetobacter baumannii* strains listed on Table 1. It was proved that the pages of the present invention are capable infecting clinically separated *Acinetobacter baumannii* having multiple resistances, wherein ψAB2 also infect two standard strains obtained from ATCC, in addition to infect clinically separated *Acinetobacter baumannii* having multiple resistances.

Example 3: Morphology of *Acinetobacter baumannii* phages under TEM

The isolated ψAB2 ($10^{12}$ PFU/ml) was dropped on formvar-coated copper grid (200 mesh copper grids), negatively stained by 2% uranyl acetate, and placed on TEM (Hitachi Company, Japan; mold: H-7500, operation condition: 80 kV). The image obtained is shown in FIG. 1.

The viral particle of ψAB2 has a head portion having a size of 60 nm and 20 faces, and a tail portion having a size of about 9-11 nm and filament structures.

Example 4: PAGE electrophoresis analysis

200 ml of AB culture solution at early stage of log phase was infected with ψAB2 (MOI being about 1.0), and incubated under aeration until AB were completely lysed. Then, the culture solution was centrifuged, the supernatant was filtered with 0.45 μm film, the filtrate was centrifuged at 18,000 rpm for 2 hours (Beckman Avanti J-251), and the precipitants obtained were viral particles of phages. Then, the precipitants were dissolved with 1.0 ml of TE buffer (10 mM Tris-HCl, pH7.0 including 1.0 mM EDTA), and then super-centrifuged at 25,000 rpm, 4°C for 2 hours, so as to purify the band of phages. The purified phages band were dialyzed to remove TE buffer, and then storage at 4°C.
The phage particles were concentrated with 20% polyethylene glycol 6000, extracted with phenol/chloroform, and then precipitated with ethanol, so as to obtain DNA of phages. The DNA was treated with ApaI, BamHI, BanII, BglII, EcoRI, EcoRV, HincII, HindIII, KpnI, MluI, PstI, PvuII, SacI, SmaI, SnaBI, SphI, SspI, StuI and XbaI, respectively, and analyzed by 0.8% and 1.0% agarose gel with pulse field electrophoresis in TAE buffer.
As shown in FIG. 2A, DNA of ψAB2 was digested by BglII, EcoRI, EcoRV, HincII, HindIII, MluI, SnaBI, SphI, SspI and XbaI. The standard molecule (M) was 1-kb plus DNA Ladder (Invitrogen, CA). Upon restriction enzyme digestion analysis, the full length of phage DNA was about 35-40 kb. The restriction enzyme map of the phage DNA is shown in FIG. 2B, in which the cutting sites of BglII, EcoRI, EcoRV, MluI and XbaI are indicated.

Example 5: SDS-PAGE analysis

The purified phage particles and the sample buffer solution (62.5 mM Tris-HCl including 5% 2-mercaptoethanol, 2% sodium dodecylsulfate, 10% glycerol and 0.01% phenol blue, pH 6.8) were mixed, heated in boiled water bath for 3 minutes, and then analyzed in 12.5% SDS-PAGE.
FIG. 3 shows protein electrophoresis patterns of ψAB2. The phage has at least 10 different protein bands in the range of 21 and 140 kDa, in which the protein of 33 kDa is the most abundant and could be the coat protein of the phage.

Example 6: sequence analysis

The Sau3A1-partial fragments (ca. 15 kb) of the phage genome was cloned to pUC18, and DNA inserts from six clones were sequenced. The sequence analysis was performed by NCBI package.
Upon DNA sequencing and alignment, the sequences of SEQ. ID NO. 1 to 4 were obtained. The sequences of SEQ ID NO. 1 and SEQ ID NO. 2 are DNA sequence encoding RNA polymerase of the *Acinetobacter baumannii* phage. The sequences of SEQ ID NO. 3 and SEQ ID NO. 4 are DNA sequence encoding the head-tail connector of the *Acinetobacter baumannii* phage.
The sequences of SEQ. ID NO. 1 to 4 were aligned with the gene database of NCBI. There is no identical or similar sequence in database as the sequences of SEQ. ID NO. 1 to 4 of the present invention.
For example, before the application is filed, the alignment result shows that the DNA sequence of SEQ ID NO. 1 has 39.4% homology with phiAB1-LKA1, 41.3% homology with phiKMV, 41.3% homology with phiPT5, 41.5% homology with phiPT2, and 41.5% homology with phiLKD16. Accordingly, the DNA sequence of SEQ ID NO. 1 has no more than 40% homology with the sequences in NCBI database.
In addition, the amino acid sequence encoded by the DNA sequence of SEQ ID NO. 1 has 30.6% homology with phiAB1-LKA1, 29.4% homology with phiKMV, 29.4% homology with phiPT5, 29.3% homology with phiPT2, and 29.2% homology with phiLKD16. Accordingly, the amino acid sequence encoded by the DNA sequence of SEQ ID NO. 1 has no more than 30% homology with the protein sequences in NCBI database.
It is known in the art that the DNA sequence of RNA polymerase is highly conserved region in viral genome. Therefore, homology among species can be determined by identifying homology of RNA polymerase. Upon sequence alignment, there is no identical or similar viral sequence as the sequences of the phages in the present invention. It is clear that the present invention provides novel phages. Further, the sequences of SEQ ID NO.1 and SEQ ID NO.2 have been registered in NCBI database, and had the registration numbers as bankit1192576 FJ809932 and bankit1192679 FJ809933, respectively (which are not published before the filing of the present application).

Example 7: efficiency of sterilization

The AB culture (host cell) was incubated to $OD_{600}$ as 0.6 U, and then the *Acinetobacter baumannii* phage was added to the host cell culture (MOI: 0.0005) and incubated at room temperature. At the time points of 0, 1, 2, 3, 4, 5, 10, 20 and 30 min, 100 μl of culture was sampled and diluted with 0.9 ml of cold LB, and then centrifuged at 12,000x g for 5 minutes. The supernatant was collected, and the amount of the phage without attaching to host cells was determined. For example, the result of ψAB2 to ATCC 17978 is shown in FIG. 4.
Upon observation of the host cell culture added with the phages, the culture solution turned from turbid into clear in 100 minutes. It is proved that the host cells were all lysed, and thus the composition of the present invention can be used for sterilization.

As shown in FIG. 4, about 75% of the phage particles attached to the host cells in 2 minutes, about 95% of the phage particles attached to the host cells in 4 minutes, and 100% of the phage particle attached to the host cells in 10 minutes.

Further, the replication curve of the phages was determined by one-step growth curve. The AB culture solution ($OD_{600}$: 0.8U) was centrifuged, and the precipitant was collected and resuspended in 0.8 ml of LB medium to a concentration of $10^9$ CFU/ml. The AB-specific phages (MOI: 0.0001) were added to the host cell culture solution, and placed at 4°C for 30 minutes, such that the phages attached to the host cells. The mixture was centrifuged at 12,000x g for 10 minutes, and the precipitant including the infected bacteria was re-suspended with 20 ml of LB medium, and incubated at 37°C. The culture was sampled every 5 minutes, and the samples were immediately diluted and quantified. For example, the result of ψAB2 to ATCC 17978 is shown in FIG. 5.

The definition of a latent period is from the attachment (excluding 10 minutes of the pretreatment) to the beginning of the first burst (bacteria were lysed, and phages were released). As shown in FIG. 5, the latent period is 15 minutes. The ratio of the amount of phage particles to the initial amount of the infected bacteria was calculated. The average burst is about 200 PFU/cell.

Similarly, the infectivity of ψAB1 to ψAB4 was determined. The results showed that all the phages ψAB1 to ψAB4 had strong infectivity, short latent period, big burst and immediate sterilization.

Example 8: compatibility

The compatibility of the AB phages isolated in embodiment was determined with the surfactants TWEEN 20, TWEEN 80 and Triton X-100 (Sigma-Aldrich Biotechnology, USA). The common concentration of the conventional surfactants is 0.1~1wt%. Thus, 1 wt% of the above surfactants was mixed with the AB phages ($5x10^7$ PFU/ml). The mixture was incubated at room temperature, and the concentration of phage culture was determined every 24 hours. The viability of phages was calculated based on the following equation, so as to determine the effects of surfactants on the phages.

$$\text{viability of phages} = \text{concentration of sampled phage culture/original concentration of phage culture}$$

Upon determination, the activities of ψAB1 to ψAB4 phages were not influenced by 0.1~1 wt% of surfactants. For example, FIG. 6 shows the result of ψAB2. As shown in FIG. 6, the phages had excellent stability in Triton X-100 and TWEEN 20, and moreover, the phages had varied viability in TWEEN 80 but still had infectivity to host cells. As shown in FIG. 6, the concentration of phages was decreased slightly and then increased gradually. It was known that by using coefficient variation, CV values of the three surfactants were all less than 20%. Accordingly, the phages were very stable in these surfactants, and all had bioactivity.

Hence, at least one of ψAB1 to ψAB4 phages can be mixed with a carrier (such as water, a surfactant (Triton X-100, TWEEN 20, TWEEN 80, etc.) to form a composition for sterilization of equipments or environment. Preferably, in the composition, the initial content of the phage is $1x10^7$ to $1x10^9$ PFU/ml, and the content of the surfactant is 0.1 to 2 wt%.

Example 9: bioactivity of the phages isolated in example 1 under different conditions

1. Temperature

The phages were diluted with autoclaved water to $10^8$ PFU/ml, and then placed at different temperatures, 4°C, 25°C, 37°C, 42°C, -20°C and -80°C. For the tests at 4°C, 25°C and 37°C, the concentration of the phage culture was determined every 3 hours in 24 hours, and then determined every week for 12 weeks. As shown in FIG. 7A, there were respective two groups at -20°C and -80°C, in which one group was repeatedly frozen and thawed and the determination was performed for 12 weeks, and the other group was thawed once and the determination was performed for 5 weeks. The results were shown in FIG. 7B.

2. pH

The phages were diluted with acidic solution (pH 4) or basic solution (pH 11) to$10^8$ PFU/ml. The concentration of the phage cultures at pH 4.7, 7 and 11 was determined every 3 hours in 24 hours, and then determined every week for 12 weeks. FIG. 8 shows the results.

3. Chemicals

The phages were added to chloroform solution (0.5% and 2%, respectively), and the phages were diluted to $10^8$ PFU/ml. The concentration of the phage culture was determined every 3 hours in 24 hours. Then, the concentration of the phage culture in 0.5% chloroform solution was determined every week for 3 weeks, and the concentration of the phage culture in 2% chloroform solution was determined every week for 6 weeks. FIG. 9 shows the results.

4. Dry treatment

$10^{10}$ PFU/ml of phages were grouped into groups A and B. Groups A and B of the phages were diluted with peptone ad autoclaved water, respectively, for ten folds, and then dried in the speed vac system. After dry treatment, groups A and B of the phages were respectively dissolved in 0.5 ml of peptone and 0.5 ml of autoclaved water. The concentrations of the phages before and after the dry treatment were determined and shown in Table 2.

Table 2

| | Average concentration of phages after dry treatment (PFU/ml) | Original concentration of phages (PFU/ml) | Viability of re-dissolved phages |
|---|---|---|---|
| Group A | $2.18\times10^9$ | $1.02\times10^{10}$ | 21.3% |
| Group B | $2.30\times10^9$ | $1.02\times10^{10}$ | 33.4% |

According to the above results, the phages of the present invention survived for at least 8 weeks at low temperatures (-20°C, -80°C, 4°C), and had the viability more than 5%. At 25°C and 37°C, the phages survived for at least 11 weeks and had the viability more than 14.9%. At 42°C for 2 weeks, the phages had the viability as 14.8%. The phages of the present invention incubated at pH 11 for about 11 weeks had the viability as about 30%. There were alive phages, which were incubated at pH 4 for 11 weeks. In addition, the phages of the present invention in 0.5% and 2% chloroform solution survived for at least 3 weeks and had the viability as 30%. After dry treatment and re-dissolution, the viability of phages was more than 20%.

Accordingly, the phages of the present invention have tolerance to temperatures, humidity, pH and chemicals, and maintain good viability in these conditions.

## Claims

1. An isolated lytic phage specific for *Acinetobacter baumannii,*

   - being DSM 23599 phage, wherein the DSM 23599 phage is deposited in DSMZ (German Collection of Microorganisms and Cell Cultures, Germany) and has a deposition number DSM 23599, or
   - being selected from the group consisting of DSM 23600 phage and variants of the DSM 23600 phage, wherein the DSM 23600 phage is deposited in DSMZ (German Collection of Microorganisms and Cell Cultures, Germany) and has a deposition number DSM 23600, and wherein each of the variants of the DSM 23600 phage has one or more sequences selected from the group consisting of sequences having more than 80% homology to

     SEQ ID NO: 1 and 2 and encoding an RNA polymerase of the Acinetobacter baumannii phage, and
     SEQ ID NO: 3 and 4 and encoding a head-tail connector of the Acinetobacter baumannii phage,

   and has a genomic sequence with a homology of more than 80% compared to the genomic sequence of the DSM 23600 phage.

2. The isolated lytic phage specific for *Acinetobacter baumannii* of claim 1, having bioactivity at pH 4 to 12.

3. The isolated lytic phage specific for *Acinetobacter baumannii* of any of the preceding claims, having bioactivity in a surfactant.

4. The isolated lytic phage specific for *Acinetobacter baumannii* of claim 3, wherein the surfactant is one selected from the group consisting of an anionic surfactant, a cationic surfactant, an amphoteric surfactant and a non-ionic surfactant.

5. The isolated lytic phage specific for *Acinetobacter baumannii* of claim 4, wherein the surfactant is a non-ionic surfactant.

## Patentansprüche

1. Isolierter lytischer Phage, der für *Acinetobacter baumannii* spezifisch ist,

   - bei welchem es sich um den Phagen DSM 23599 handelt, wobei der Phage DSM 23599 bei der DSMZ (Deutsche Sammlung von Mikroorganismen und Zellkulturen, Deutschland) hinterlegt ist und die Hinterlegungsnummer DSM 23599 besitzt, oder
   - welcher aus der Gruppe, bestehend aus dem Phagen DSM 23600 und Varianten des Phagen DSM 23600, ausgewählt ist, wobei der Phage DSM 23600 bei der DSMZ (Deutsche Sammlung von Mikroorganismen und Zellkulturen, Deutschland) hinterlegt ist und die Hinterlegungsnummer DSM 23600 besitzt, und wobei jede der Varianten des Phagen DSM 23600 eine oder mehrere Sequenzen aufweist, ausgewählt aus der Gruppe bestehend aus Sequenzen, die mehr als 80% Homologie zu

     SEQ ID NO: 1 und 2 aufweisen und eine RNA-Polymerase des Acinetobacter-baumannii-Phagen codieren, und zu
     SEQ ID NO: 3 und 4 aufweisen und einen Kopf-Schwanz-Konnektor des Acinetobacter-baumannii-Phagen codieren,

   und eine genomische Sequenz mit einer Homologie von mehr als 80% im Vergleich zur genomischen Sequenz des Phagen DSM 23600 aufweist.

2. Isolierter lytischer Phage, der für *Acinetobacter baumannii* spezifisch ist, nach Anspruch 1, der Bioaktivität bei pH 4 bis 12 aufweist.

3. Isolierter lytischer Phage, der für *Acinetobacter baumannii* spezifisch ist, nach einem der vorstehenden Ansprüche, der Bioaktivität in einem Tensid aufweist.

4. Isolierter lytischer Phage, der für *Acinetobacter baumannii* spezifisch ist, nach Anspruch 3, wobei das Tensid ausgewählt ist aus der Gruppe bestehend aus einem anionischen Tensid, einem kationischen Tensid, einem amphoteren Tensid und einem nicht-ionischen Tensid.

**5.** Isolierter lytischer Phage, der für *Acinetobacter baumannii* spezifisch ist, nach Anspruch 4, wobei das Tensid ein nicht-ionisches Tensid ist.

## Revendications

**1.** Phage lytique isolé spécifique au *Acinetobacter Baumanii,*

- étant le phage DSM 23 599, dans lequel le phage DSM 23 599 est déposé auprès de la DSMZ (Deutsche Sammlung von Mikroorganismen und Zellkulturen, Allemagne) et présente un numéro de dépôt DSM 23 599, ou
- étant sélectionné parmi le groupe consistant en le phage DSM 23600 et les variants du phage DSM 23600, dans lequel le phage DSM 23600 est déposé auprès de la DSMZ (Deutsche Sammlung von Mikroorganismen und Zellkulturen, Allemagne) et présente un numéro de dépôt DSM 23600, et dans lequel chacun des variants du phage DSM 23600 présente une ou plusieurs séquences sélectionnées parmi le groupe consistant en des séquences présentant plus de 80% d'homologie avec :

SEQ IN N° : 1 et 2 et codant une ARN polymérase du phage Acinetobacter Baumanii, et
SEQ IN N° : 3 et 4 et codant un connecteur tête-bêche du phage Acinetobacter Baumanii,

et présente une séquence génomique avec une homologie supérieure à 80% par comparaison avec la séquence génomique du phage DSM 23600.

**2.** Phage lytique isolé spécifique au *Acinetobacter Baumanii* selon la revendication 1, présentant une bioactivité à un pH de 4 à 12.

**3.** Phage lytique isolé spécifique au *Acinetobacter Baumanii* selon l'une quelconque des revendications précédentes, présentant une bioactivité dans un surfactant.

**4.** Phage lytique isolé spécifique au *Acinetobacter Baumanii* selon la revendication 3, dans lequel le surfactant est un élément sélectionné parmi le groupe consistant en un surfactant anionique, un surfactant cationique, un surfactant amphotère et un surfactant non ionique.

**5.** Phage lytique isolé spécifique au *Acinetobacter Baumanii* selon la revendication 4, dans lequel le surfactant est un surfactant non ionique.

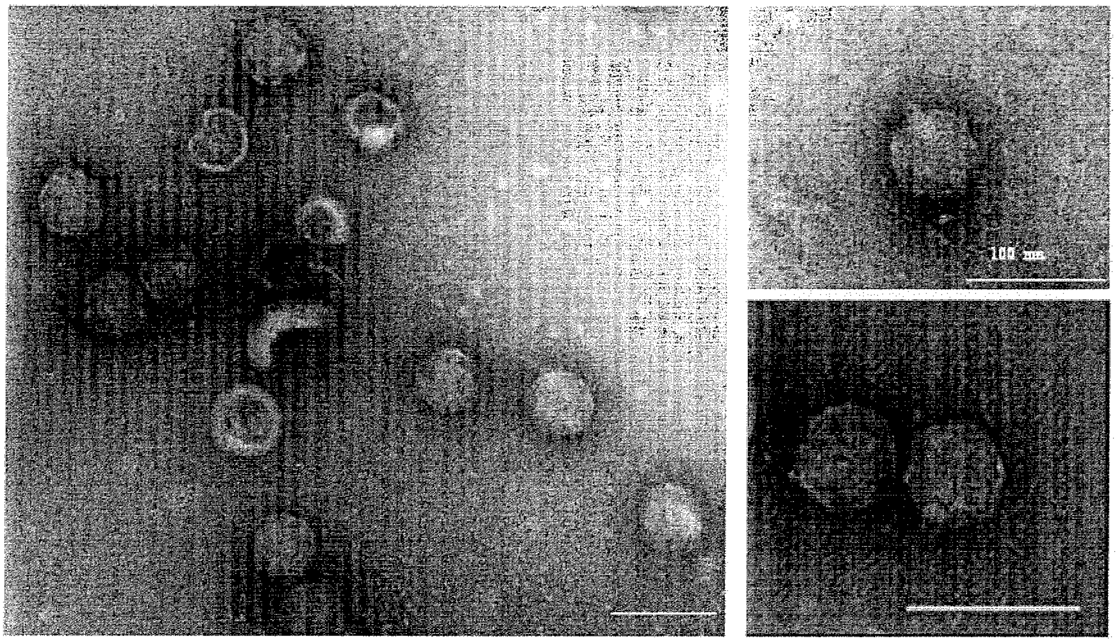


FIG.1

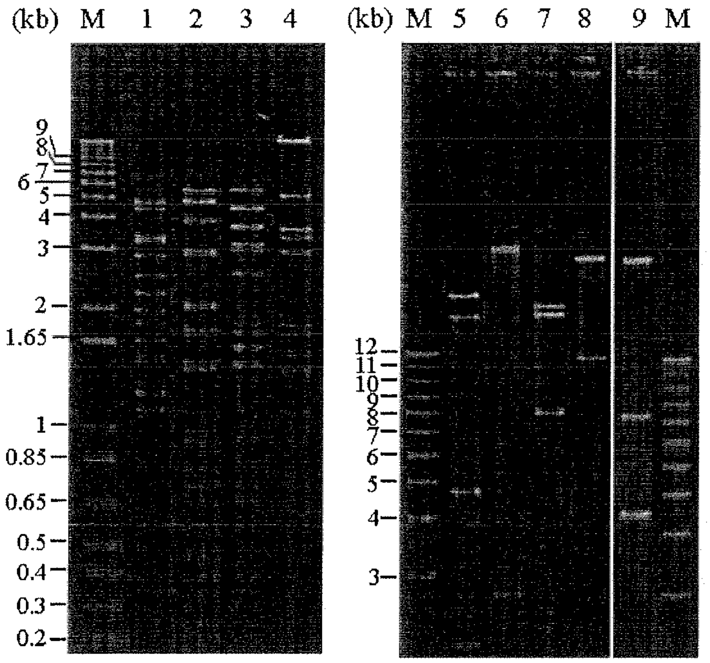
(kb) M 1 2 3 4
(kb) M 5 6 7 8 9 M
9
8
7
6
5
4
3
2
1.65
1
0.85
0.65
0.5
0.4
0.3
0.2
12
11
10
9
8
7
6
5
4
3

FIG.2A

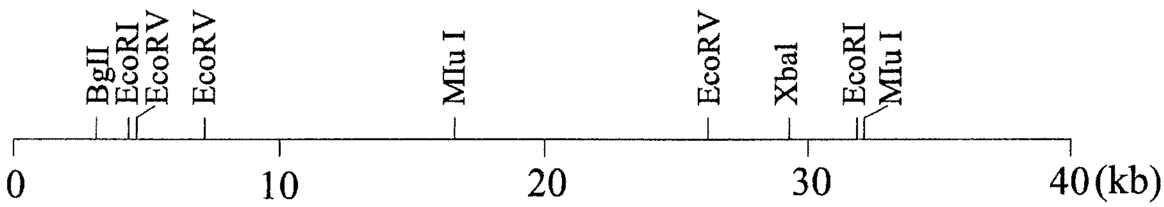
BglI
EcoRI
EcoRV
EcoRV
Mlu I
EcoRV
XbaI
EcoRI
Mlu I
0
10
20
30
40 (kb)

FIG.2B

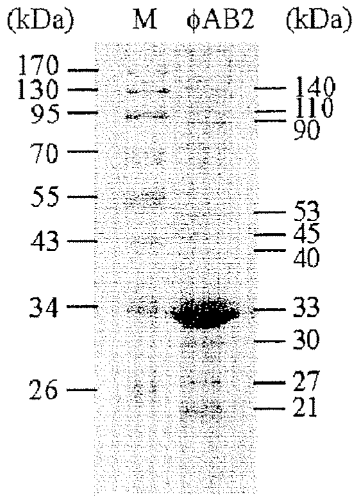
(kDa)
M
φAB2
(kDa)
170
130
95
70
55
43
34
26
140
110
90
53
45
40
33
30
27
21

FIG.3

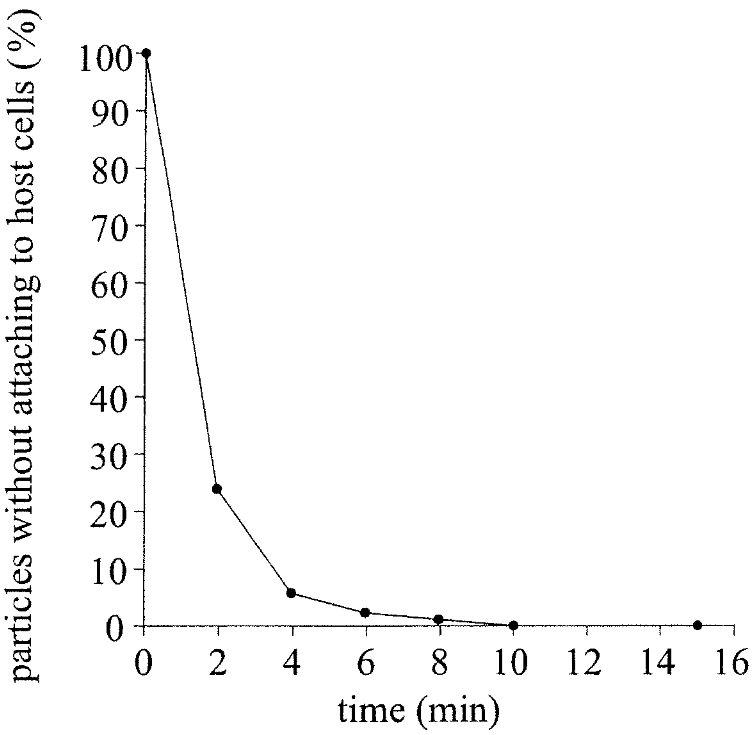
particles without attaching to host cells (%)
100
90
80
70
60
50
40
30
20
10
0
0 2 4 6 8 10 12 14 16
time (min)

FIG.4

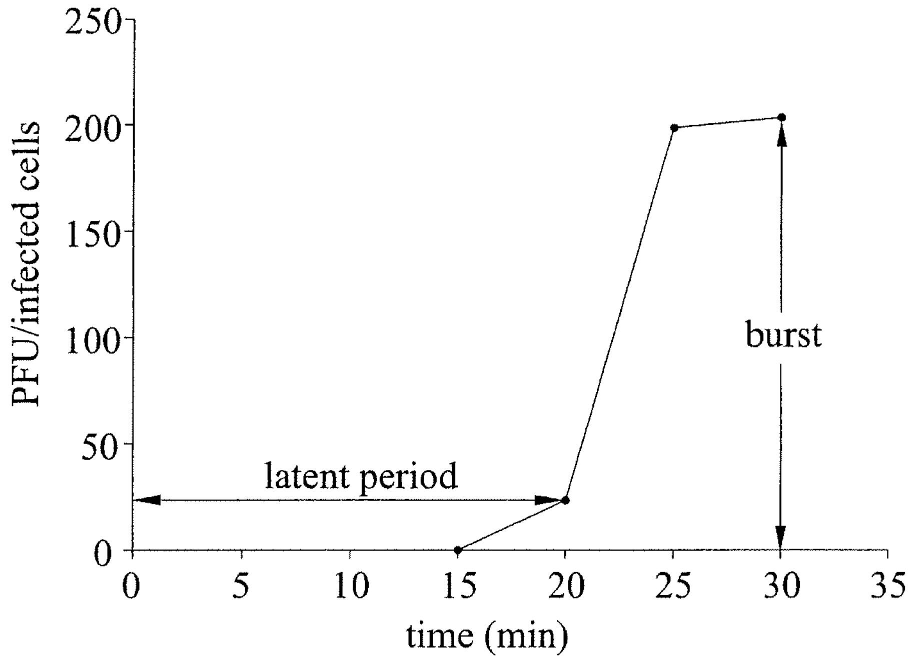
PFU/infected cells
250
200
150
100
50
0
0 5 10 15 20 25 30 35
time (min)
latent period
burst

FIG.5

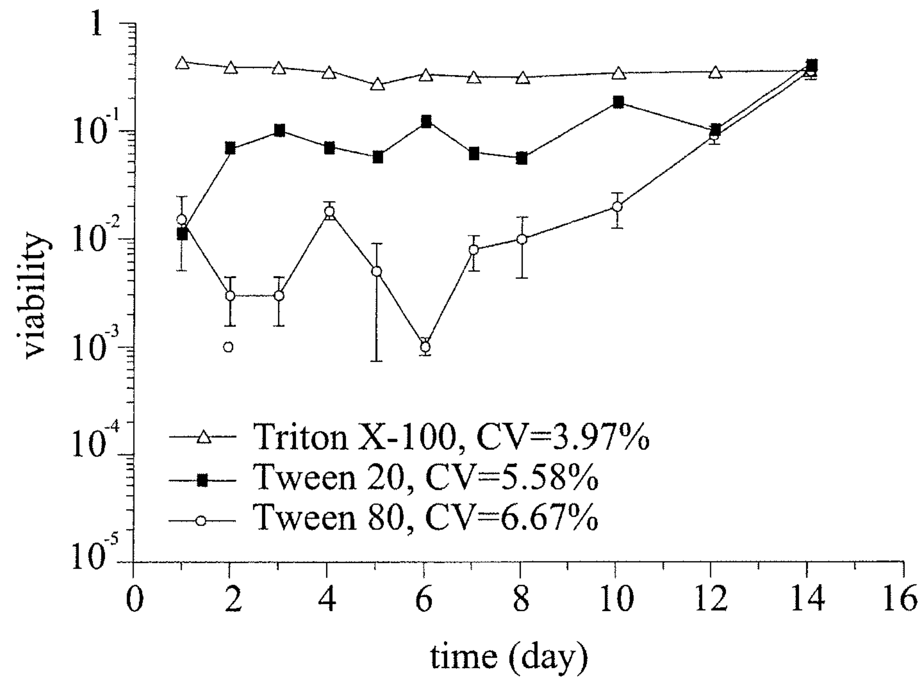
1
$10^{-1}$
$10^{-2}$
$10^{-3}$
$10^{-4}$
$10^{-5}$
viability
0
2
4
6
8
10
12
14
16
time (day)
Triton X-100, CV=3.97%
Tween 20, CV=5.58%
Tween 80, CV=6.67%

FIG.6

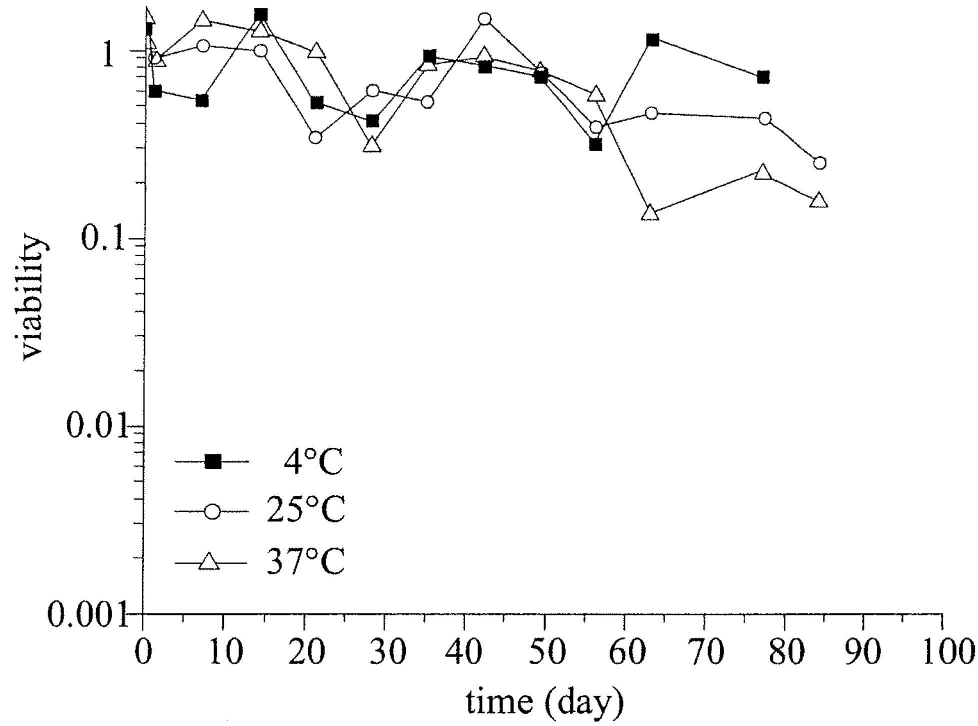
viability
1
0.1
0.01
0.001
4°C
25°C
37°C
0
10
20
30
40
50
60
70
80
90
100
time (day)

FIG.7A

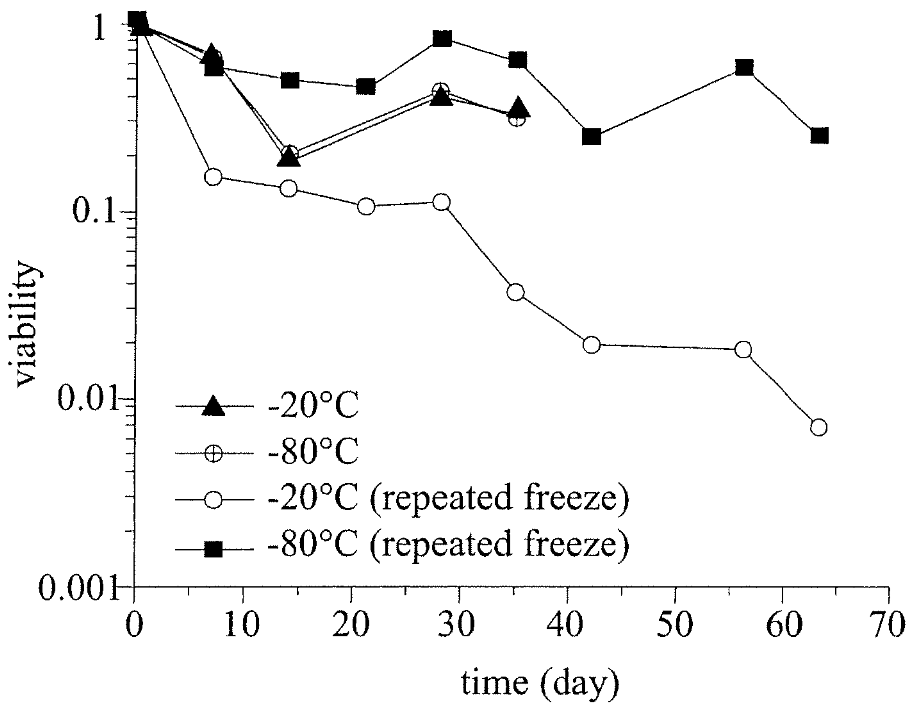
viability
1
0.1
0.01
0.001
-20°C
-80°C
-20°C (repeated freeze)
-80°C (repeated freeze)
0
10
20
30
40
50
60
70
time (day)

FIG.7B

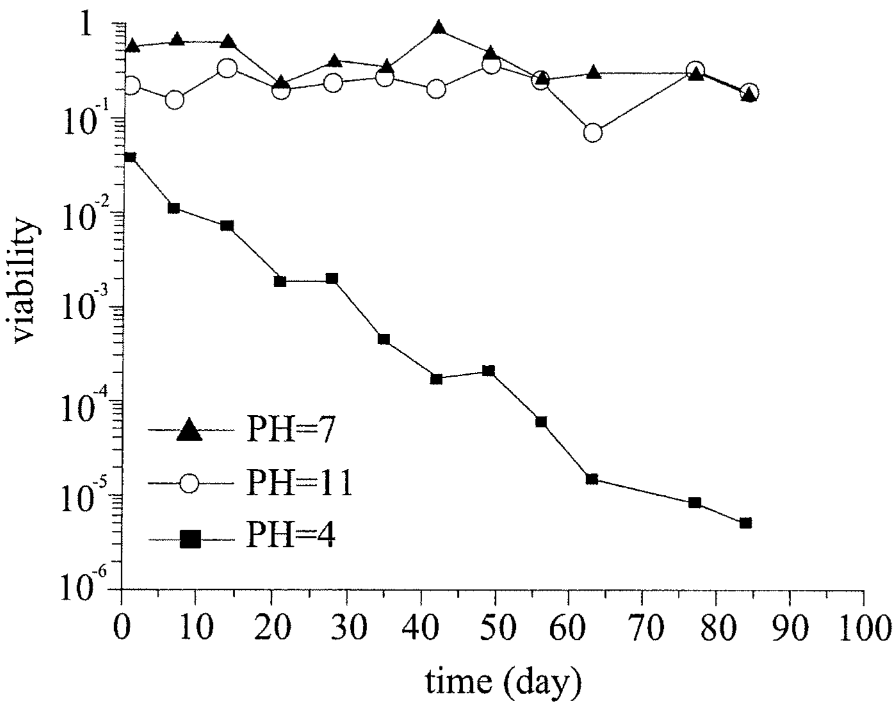

1
$10^{-1}$
$10^{-2}$
$10^{-3}$
$10^{-4}$
$10^{-5}$
$10^{-6}$
viability
PH=7
PH=11
PH=4
0 10 20 30 40 50 60 70 80 90 100
time (day)


FIG.8

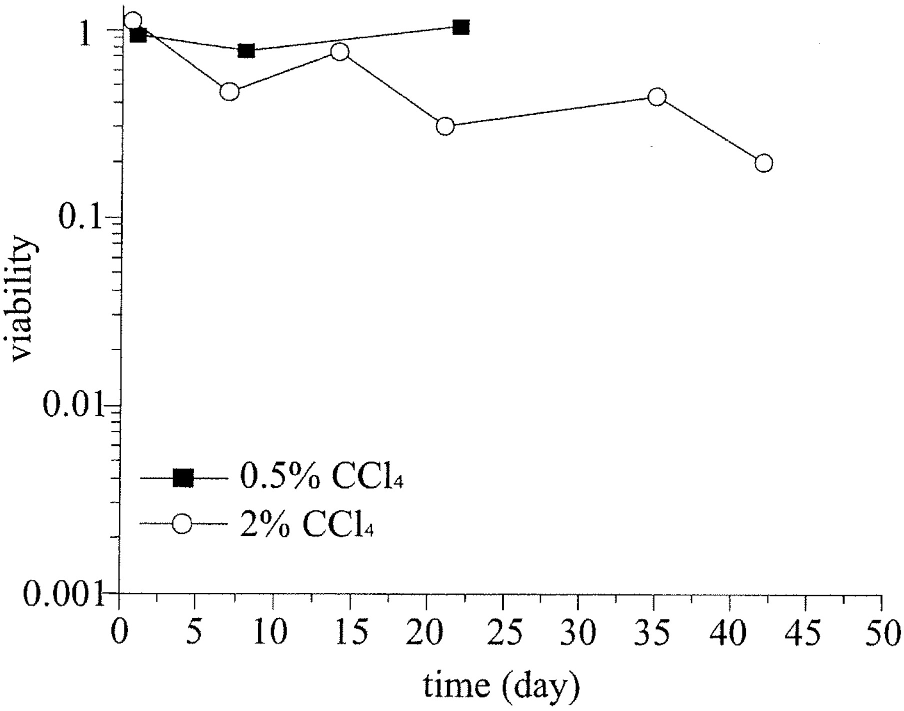

1
0.1
0.01
0.001
viability
0.5% $CCl_4$
2% $CCl_4$
0 5 10 15 20 25 30 35 40 45 50
time (day)


FIG.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5688501 A
- US 5997862 A
- US 6248324 B
- US 6485902 B
- US 6121036 A
- US 6699701 B


### Non-patent literature cited in the description


- **SHEN et al.** discloses isolation and characterization of lytic phages against pandrug resistant Acinetobacter baumannii. *Abstracts of the General Meeting of the American Society for Microbiology,* 2006, vol. 106, 369